# EUROPEAN PATENT APPLICATION

(11) **EP 1 559 777 A1**
(43) Date of publication of application: **03.08.2005**
(21) Application number: 05075598.2
(22) Date of filing: 12.04.2002
(51) Int. Cl.: C12N 5/06, G01N 33/50, C12Q 1/68, A61K 45/00

(54) **Process for maturating small heptocyte-rich colonies into liver tissue**

(30) Priority: 24.04.2001 JP 2001125525
(62) Divisional of application: 02718561.0
(71) Applicant: Hokkaido Technology Licensing Office Co., Ltd., Sapporo-Shi, Hokkaido 060-0807 (JP)
(72) Inventor: MItaka, Toshihiro, Sapporo-Shi Hokkaido 004-0863 (JP); Sugimoto, Shinichi, Izumo-shi Shimane 693-0062 (JP)
(74) Representative: Hallybone, Huw George

(57) **Abstract**

The present invention provides a process for preparing a transplantable liver tissue, cell colonies suitable for the process and a process for preparing them. At least about 70% of the cells in the cell small hepatocyte-rich colonies of the present invention are small hepatocytes. In particular, each colony comprises about 10 to 30 cells and the number of the small hepatocytes is at least about 70% based on the total number of cells in the colony. A process for preparing such small hepatocyte rich colonies is also disclosed. The present invention also discloses a process for inducing the maturation of the cell small hepatocyte-rich colonies into a liver tissue and a method of estimating the effect of a drug, particularly the effect thereof connected with normal liver function, *in vitro* using the cell colonies enriched with the small hepatocytes which has been induced to be matured.

## Description

### Background of the invention

The present invention relates to a small hepatocyte colony suitable to be induced to a liver tissue *in vitro,* a process for producing the colony and a process for efficiently inducing a liver tissue from the small hepatocyte colony.

Hepatic dysfunction of human beings is caused by various diseases such as hepatitis, hepatic cirrhosis and liver cancer. Because artificial liver has not been yet in the practical stage, the ultimate treatment of such diseases must rely on a transplantation of the liver at present. In addition, though there are numerous patients who require the transplantation of the liver not only in Japan but also in many countries in the world, the number of the donors for donating the organ is only 10 % of the required number. Thus, it is demanded to develop a process for producing a liver tissue which is usable for liver transplantation *in vitro,* a process for preparing colonies of precursor cell for liver tissue usable for the process and a process for producing them *in vitro.*

On the other hand, although the liver is known to be an organ having a high regenerative capacity *in vivo,* it cannot be said that the regeneration of the liver has been succeeded *in vitro*. Currently, among the cells directly constituting the liver, only the hepatocytes, biliary epithelial cells, etc. can be separately grown at cell culture level. As a method for preparing a liver tissue which can be transplanted, investigations are made on the organ culture and processes wherein ES cells are used. However, it has not yet been succeeded in forming an organ having a sufficient size by organ culture and also in producing all the cells constituting the liver from ES cells. ln particular, although ES cells are hopeful, ES cells must be prepared from individual patients and, in addition, each type of cells constituting the liver must be grown of which methods have not yet been established.

In addition to those investigations, it has been elucidated that various drug-metabolizing enzymes are present in the hepatocytes. The drug-metabolizing enzymes act on external and internal chemical substances (hereinafter referred to as "drugs") such as carcinogens, food additives, insecticides, steroids, eicosanoids such as prostaglandins, substances having a low polarity, particularly relatively lipophilic substances such as lipids or lipophilic vitamins to increase the polarity of those substances by a reaction such as hydroxylation, and then return the substances into blood. The substances will be excreted in the form of urine or they are excreted in the bile and thereby removed from the body. The drug-metabolizing enzymes thus concern with the process for the excretion from the body. In this process, two phases, i. e. phase I and phase II, or two steps are known. Phase I is a reaction for adding a substituent, mainly hydroxyl group, which is capable of increasing the polarity. This reaction is mainly catalyzed by an enzyme called cytochrome P450 (monooxygenase or CYP), for which about 150 isozymes are known. Those isozymes are specific to the structure of the substances on which they are reactive. Typical known isozymes are, for example, CYP1A1, CYP2B1, CYP3A2, CYP2E1 and CYP4A1. It is generally thought that about 50 % of drugs taken by human beings are metabolized by cytochrome P450 enzymes. Phase II is a process where a specified molecule is added to metabolites which has been modified by hydroxylation, etc. in phase I, to increase the solubility thereof in water or to facilitate the excretion into the bile. Enzymes concerning with this phase include enzymes capable of adding glucuronic acid, sulfuric acid, acetates, glutathione and amino acids and also methylation enzymes. Examples of the enzymes include glutathione S-transferase (GST), γ- glutamyl transferase (GGT), glucuronosyltransferase, acetyltransferase and ethyltransferase. Those drug-metabolizing enzymes including cytochrome P450 are generally considered to be concerned with so-called "detoxification". ln some cases, the drug-metabolizing enzymes act on specified substances to form mutagens, and they may have adverse effects such as the involvement in the generation of mutagens by reacting with particular substances or the reduction in the therapeutic effects of these drugs on each other are exerted when two or more drugs are administered together.

Among those drug-metabolizing enzymes, P450 is considered to be particularly important from the viewpoint of clinical medicine. For example, it is known that warfarin used as an anticoagulant induces the production of CYP2C9. Warfarin per se will be metabolized by a drug-metabolizing enzyme induced by itself. However, when both warfarin and phenobarbital are administered to a patient having an epileptic fit, endoplasmic reticulum hyperplasia caused by phenobarbital occurs to increase CYP2C9 and, therefore, the pharmacological effect of warfarin is further weakened. Thus, for obtaining a clinical effect of warfarin, the dose thereof must be increased to a level higher than that of the dose of warfarin administered alone. On the other hand, when the administration of phenobarbital is suddenly stopped, the metabolism of warfarin is lowered to increase the concentration thereof in blood and, as a result, the patient is apt to have hemorrhage. Further, such a phenomenon is known that when pentobarbital, a hypnotic drug, is administered after the administration of antidepressant imipramine or the like, the metabolism of pentobarbital is controlled and, as a result, the reaction time thereof becomes longer than that required when pentobarbital is administered alone. This phenomenon is caused because imipramine represses the induction of the expression of drug-metabolizing enzyme genes. It is also known that CYP2E1 is induced when ethanol is taken. This enzyme may metabolize substances contained in tobacco fumes and some kinds of solvents and may produce carcinogenicity. Thus, it is generally considered that heavy smokers who habitually drink alcohols tend to have a high carcinogenicity. Further, it is known that CYP4501A1 metabolizes aromatic hydrocarbon compounds such as 3-methylcholanthrene to form a substance reactive with DNA and thereby to produce carcinogenicity. Therefore, the induction or non-induction of various isozymes of cytochrome P450 is considered to be one of the important items to be examined in, for example, the hepatotoxicity tests of new medicines.

Tests for examining the effects of medicines on the liver function including the hepatotoxicity tests are conducted both *in vivo* and *in vitro.* It was pointed out that in the *in vivo* tests, the use and control of laboratory animals are required and there is a problem of the organ specificity. Other problems were also pointed out in the *in vivo* tests that the reproducibility and quantification are insufficient, that because cultured cells or cultured tissues are lacking in the drug-metabolizing enzyme system, a complicated system is required or in some cases, the tests *per se* cannot be conducted. Thus, a stable *in vitro* system usable for examining the effect of a drug in the liver, particularly the effect thereof associated with the liver function, has not yet been established.

The inventors reported that a certain kind of cells having a high proliferating capacity like stem cells while maintaining the function of the hepatocytes are present in the adult liver, and designated those cells as "small hepatocytes" (Mitaka T. et al., Hepatology, 16, 1992, 440-447). The inventors suggested that when the small hepatocytes are cultured in a culture medium containing fetal bovine serum, nicotinamide, EGF, etc., a monolayer colony is initially formed and then the colony will be surrounded by non-parenchymal cells such as liver epithelial cells and stellate cells. The inventors reported that when the culture is further continued, glutamine synthase and carbamoylphosphate synthase are expressed and also mitochondria, peroxisomes and glycogen granules become to be possible to be microscopically observed. However, a process for preparing small hepatocytes or for preparing a transplantable liver tissue or a process for inducing the liver tissue has not yet been developed.

### Summary of the Invention

The object of the present invention is to provide a process for preparing a transplantable liver tissue, cell colonies suitable for the process and a process for preparing the cell colonies. ln particular, the present invention provides small hepatocyte colonies suitable for preparing a transplantable liver tissue, a process for preparing them and a process for inducing the liver tissue from the small hepatocyte colonies.

Another object of the present invention is to provide a process for estimating the effects of drugs, particularly the effects associated with normal liver function *in vitro.*

The present invention provides a cell colony enriched in small hepatocytes wherein at least about 70 % in number of the total cells are small hepatocytes, in particular, small hepatocyte-rich colonies each of which is composed of about 10 to 30 cells and wherein at least about 70 % in number of the total cells are small hepatocytes.

The present invention also provides a process for preparing small hepatocyte-rich colonies and suitable for preparing a transplantable liver tissue, wherein small hepatocytes isolated from the liver or being successively subcultured are recovered in the form of cell colonies at the early stage of the culture. In particular, the present invention provides a process for preparing small hepatocyte-rich colonies and suitable for preparing a transplantable liver tissue, where the cell colonies are non-enzymatically isolated wherein each cell colony is composed of 10 to 30 cells including small hepatocytes which have been isolated from the liver or successively subcultured.

Namely, the present invention provides a process for preparing small hepatocyte-rich colonies, which comprises the steps of:
(i) isolating hepatocytes from a liver,
(ii) fractionating the separated hepatocytes into a heavy fraction enriched with parenchymal cells and a light fraction enriched with non-parenchymal cells but having a low parenchymal cell content, and recovering the light fraction,
(iii) culturing the cells in the light fraction in a culture medium containing nicotinamide to form small hepatocyte colonies, and
(iv) recovering the small hepatocyte colonies each containing 10 to 30 cells in total.

The present invention also provides a process for preparing small hepatocyte-rich colonies, which comprises the steps of:
(i) detaching colonies from a culture dish after reacting or without reacting an enzyme on small hepatocytes forming the colonies,
(ii) successively subculturing the recovered small hepatocytes by using a culture medium containing nicotinamide to form colonies composed of 10 to 30 cells in total, and
(iii) recovering the colonies each composed of 10 to 30 cells in total.

Further, the present invention provides a cell colony prepared as described above, wherein the number of the small hepatocytes is at least about 70 % based on the total cells.

The present invention provides a process for inducing a liver tissue from the small hepatocyte colonies by culturing the small hepatocyte-rich colonies isolated as described above, adding an extracellular matrix thereto, culturing them for a given period of time and further culturing them in an extracellular matrix-free medium.

The present invention provides a process for inducing a liver tissue from the small hepatocyte colonies by transplanting the small hepatocyte colonies isolated as described above onto a sheet of a biocompatible material and further culturing them for a given period of time.

The present invention provides a method of estimating an effect of a chemical substance on the liver function *in vitro* by contacting the small hepatocyte-rich colonies matured as described above with the chemical substance and identifying a gene the expression of which is induced or repressed in the cells in the above-described small hepatocyte-rich colonies and/or determining the expression level thereof.

ln particular, the present invention provides a method of estimating an effect of a chemical substance on the liver function *in vitro* by contacting the small hepatocyte-rich colonies matured as described above with the chemical substance and identifying a drug-metabolizing enzyme gene the expression of which is induced or repressed in the cells in the above-described small hepatocyte-rich colonies and/or determining the expression level thereof.

The present invention provides a method of determining whether a chemical substance induces or represses the expression of a drug-metabolizing enzyme in the liver of a living body by contacting the small hepatocyte-rich colonies matured as described above with the chemical substance and determining the capacity of the chemical substance to induce or repress the expression of the drug-metabolizing enzyme gene in the cells in the small hepatocyte-rich colonies.

The present invention provides a method which comprises the steps of contacting the small hepatocyte-rich colonies matured as described above with a chemical substance, identifying a gene the expression of which is induced or repressed in the cells in the above-described small hepatocyte-rich colonies, and/or determining the expression level thereof, comparing the induction or repression pattern of the gene the expression of which is induced or repressed with an induction or repression pattern of an expression of the gene associated with a chemical substance having a known effect, and estimating the effect, on the liver function *in vitro*, of the chemical substance which exhibits the induction or repression pattern similar to the pattern for the chemical substance having the known effect as the effect of the chemical substance placed in contact with the small hepatocyte-rich colonies.

In particular, the present invention provides a method which comprises the steps of contacting small hepatocyte-rich colonies matured as described above with a chemical substance, identifying a drug-metabolizing enzyme gene the expression of which is induced or repressed in the cells in the above-described small hepatocyte-rich colonies and/or determining the expression level thereof, comparing the induction or repression pattern of the drug-metabolizing enzyme gene the expression of which is induced or repressed with an induction or repression pattern for the drug-metabolizing enzyme gene associated with a chemical substance having a known effect, and estimating the effect, on the liver function *in vitro,* of the chemical substance which exhibits the induction or repression pattern similar to the pattern of the chemical substance having a known effect, as the effect of the chemical substance placed in contact with the small hepatocyte-rich colonies.

Specifically, the present invention provides the above-described processes for estimating the effect of chemical substances, wherein drug-metabolizing enzymes are cytochrome P450 enzymes.

### Brief Description of the Drawings

Fig. 1 shows the results of the determination of the area of adhering colonies which was detached and transferred to a new culture medium, as observed with a phase-contrast microscope. A mark was put on a culture dish and the colonies were identified using the mark as the index. The average and the standard deviation of the data obtained from 6 culture dishes are shown in Fig. 1.
Fig. 2 is a graph showing the number of cells constituting a colony. The number of the cells immunostained with cytokeratin 8, a hepatocyte marker, was counted under a microscope to obtain the results shown in Fig. 2. Three culture dishes were used for one experiment. The average and the standard deviation of the data obtained by two experiments are shown in Fig. 2.
Fig. 3 shows the induction of expression of C/EBPα protein with Matrigel. MH represents mature hepatocytes, 0 (day) represents before the start of the treatment, C represents control (without Matrigel), and M represents the addition of Matrigel (500 µg/ml).
Fig. 4 shows the induction of the expression of C/EBPβ protein with Matrigel. MH represents mature hepatocytes, 0 (day) represents before the start of the treatment, C represents control (without Matrigel), and M represents the addition of Matrigel (500 µg/ml).
Fig. 5 shows the induction of the expression of HNF4α protein with Matrigel. MH represents mature hepatocytes, 0 (day) represents before the start of the treatment, C represents control (without Matrigel), and M represents the addition of Matrigel (500 µg/ml).
Fig. 6 shows the induction of the expression of HNF6 protein with Matrigel. MH represents mature hepatocytes, 0 (day) represents before the start of the treatment, C represents control (without Matrigel), and M represents the addition of Matrigel (500 µg/ml).
Fig. 7 shows the amount of albumin secreted into the culture medium. N represents the serum or plasma of a normal rat, C represents control (without Matrigel), and M represents the addition of Matrigel (500 µg/ml).
Fig. 8 shows the amount of transferrin secreted into the culture medium. N represents the serum or plasma of a normal rat, C represents control (without Matrigel), and M represents the addition of Matrigel (500 µg/ml).
Fig. 9 shows the amount of α1-antitrypsin secreted into the culture medium. N represents the serum or plasma of a normal rat, C represents control (without Matrigel), and M represents the addition of Matrigel (500 µg/ml).
Fig. 10 shows the amount of fibrinogen secreted into the culture medium. N represents the serum or plasma of a normal rat, C represents control (without Matrigel), and M represents the addition of Matrigel (500 µg/ml).
Fig. 11 shows the change in amount of albumin secreted into the culture medium with time. After the separation of the colonies and the seeding, followed by the culture for 11 days, the addition of Matrigel (500 µg/ml) for 2 days was started on the 11^{th} day, and the amount of albumin secreted by the small hepatocytes was compared with that secreted when Matrigel was not added.
Fig. 12 shows the induction of the expression of tryptophan dioxygenase protein. MH represents mature hepatocytes, 0 (day) represents before the start of the treatment, C represents control (without Matrigel), and M represents the addition of Matrigel (500 µg/ml).
Fig. 13 shows the induction of the expression of serine dehydratase protein. MH represents mature hepatocytes, 0 (day) represents before the start of the treatment, C represents control (without Matrigel), and M represents the addition of Matrigel (500 µg/ml).
Fig. 14 shows the change in amount of albumin secreted by the small hepatocytes cultured on a sheet with time. Black square(■) represents the culture on the sheet and black lozenge(◆) represents the culture on the collagen-coated culture dish (control).
Fig. 15 shows the amount of transferrin secreted in the culture medium by small hepatocytes cultured on a sheet. N represents the serum or plasma of a normal rat, C represents control (culture on a collagen-coated culture dish), and S represents the culture on the collagen sheet.
Fig. 16 shows the amount of haptoglobulin secreted by the small hepatocytes, cultured on the sheet, in the culture medium. N represents the serum or plasma of a normal rat, C represents control (culture on a collagen-coated culture dish), and S represents the culture on the collagen sheet.
Fig. 17 shows the amount of fibrinogen secreted by the small hepatocytes, cultured on the sheet, in the culture medium. N represents the serum or plasma of a normal rat, C represents control (culture on a collagen-coated culture dish), and S represents the culture on the collagen sheet.

### Description of the Preferred Embodiments

The present invention provides small hepatocyte colonies suitable for producing a transplantable liver tissue, a process for preparing them and a method of inducing a liver tissue from the small hepatocytes. Some embodiments of the present invention will be described below. The term "cell colony" or "colony" herein indicates a cell aggregate formed as result of cell growth. These terms are used irrelevantly to the number of cells constituting the colony. The term "small hepatocyte colony" indicates a colony containing small hepatocytes, and the term is used irrelevantly to the number of the cells constituting each colony or the proportion of the small hepatocytes in the colony. On the other hand, "hepatocyte-rich colony" herein indicates a cell colony containing small hepatocytes wherein at least about 70 % of the cells, based on the total number of cells constituting the cell aggregate, are the small hepatocytes.

The present invention employs the small hepatocytes. Then term "small hepatocyte" herein does not merely mean a small cell derived from a liver but it means a cell isolated from the liver by the method which will be described below or by other similar method. The small hepatocytes are the small cells of a special kind derived from the liver, which have a high proliferating capacity and a phenotype almost similar to that of mature hepatocytes as to the markers such as albumin, transferrin, cytokeratin (CK) 8 or CK 18, and they also have characteristics of hepatocytes as to the ultrastructure. These cells have been found by the inventors and the details thereof are described in Mitaka T, etc. Hepatology, 16, 440-447 (1992) and Mitaka T, Sato F, Mizuguchi T., etc., Hepatology, 29, 111-135 (1999).

The small hepatocytes used in the present invention can be prepared, for example, as follows: cells from the liver can be obtained by treating the liver tissue taken from a human being or from another animal with a solution containing collagenase or the like. In this process, an ordinary method of perfusion of the liver with collagenase can be employed. The obtained cell suspension is passed through a mesh of a suitable size, if necessary, to remove undigested tissue debris and other fine tissue pieces. Although the small hepatocyte colonies can be obtained by directly culturing the cell suspension, it is preferred to remove parenchymal cells and unnecessary tissue debris as far as possible before the culture. The parenchymal cells can be removed by a low-speed centrifugation as described below. The low-speed centrifugation refers to a condition where a fraction enriched with the parenchymal cells and unnecessary tissue debris is separated from a light fraction enriched with non-parenchymal cells. Preferably, the low-speed centrifugation means a condition enough for separating a fraction mainly comprising the parenchymal cells and unnecessary tissue debris from a light fraction mainly comprising the small hepatocytes and non-parenchymal cells and substantially free from the parenchymal cells. After the fractionation of the cells from the liver, obtained by the above-described method, under those conditions, the small hepatocytes are obtained in a larger amount in the light fraction. Specifically, when the cell suspension is centrifuged at a low speed of, for example, 50 x g for one minute, it can be divided into a heavy fraction mainly containing the parenchymal cells and a light supernatant fraction mainly containing relatively light cells including non-parenchymal cells such as stellate cells, Kupffer cells and sinusoidal endothelial cells. Under such a centrifugation condition, the small hepatocytes are obtained mainly in the supernatant fraction.

The higher the degree of acceleration or the longer the centrifugation time, the lower the relative amount of the parenchymal cells in the supernatant fraction will be, however, in this case, the relative amount of the precipitated small hepatocytes is also increased. Thus, the centrifugation is preferably conducted at about 50 x g for about 1 minute or shorter. The supernatant fraction may be further repeatedly centrifuged, precipitated and suspended to remove the parenchymal cells and unnecessary tissue debris. Specifically, for example, the supernatant fraction is centrifuged at 50 x g for 5 minutes, and then the precipitate is suspended in a suitable culture medium and again centrifuged at 50 x g for 5 minutes. The resulted precipitate is suspended in a similar culture medium and again centrifuged at 150 x g for 5 minutes and the precipitated cells are suspended in a fresh culture medium. The number of the cells in the cell suspension is counted and the cell density can be adjusted to that required for the subsequent culture or treatment. The suspension is usually prepared at a cell density of 1 x 10⁵ at 5 x 10⁵ cells/ml.

The cells thus obtained can be cultured in a basal medium containing serum, nicotinamide, vitamin C, antibiotics, growth factors and other additives generally used for the cell culture, for example, Dulbecco's modified Eagle medium at 37°C. The culture medium for growing or maintaining the small hepatocytes contains nicotinamide and preferably vitamin C, growth factors, DMSO, etc. for further accelerating the colony formation. Vitamin C is usually added in the form of ascorbic acid diphosphate in a concentration of preferably 0.1 mM to 1.0 mM, more preferably 0.5 mM to 1.0 mM. Nicotinamide is used in a relatively high concentration of preferably 1 mM to 20 mM, more preferably 5 mM to 10 mM. The usable growth factors are, for example, epithelial cell growth factor (EGF), hepatocyte growth factor (HGF) and transforming growth factor α (TGFα). Among the growth factors, TGFα is particularly preferred. When TGFα is added, it is used at a concentration of 1 µg/l to 100 µg/l, preferably 5 µg/l to 50 µg/l.

During the primary culture, DMSO is added at a concentration of preferably about 0.1 to about 2 % (v/v), more preferably about 0.5 % to about 1.5 % (v/v), starting from the day 4 of the culture. For the induction to the liver tissue, it is preferable that DMSO is added at a concentration of 1 % to the same culture medium as above preferably starting from the first day of the successive subculture. Serum such as fetal bovine serum (FBS) can be added for the primary culture and successive subculture of the small hepatocytes, but is preferable that a serum-free medium is used for inducing the liver tissue to be transplanted to prevent the contamination with unnecessary protein. When a serum is used, the serum is preferably from a species of the animal to which the tissue is to be transplanted, for minimizing the rejection. The medium is exchanged nearly every other day, usually 3 times a week.

The culture container for the small hepatocytes or small hepatocyte-rich colonies in the present invention may be an ordinary culture dish used for the cell culture. Usually a culture dish coated with collagen is used for culturing adherent cells. For example, culture dishes of various sizes coated with collagen from bovine corium or rat tail are commercially available and, if necessary, those culture dishes can also be prepared. Although such a culture dish is usable for the culture of the small hepatocytes also in the present invention, a culture dish not coated with collagen is preferably used for producing the small hepatocyte colonies suitable for the formation of the liver tissue, because the smaller the amount of the extracellular matrix, the milder the condition for dissociating the cells will be, and the small hepatocytes are inclined to be preferentially detached from the container when the dish is not coated with collagen or the like. ln the present invention, the culture is preferably started at a relatively high cell density, specifically about 4 x 10⁵ to 9 x 10⁵ cells, in a 60 mm culture dish. An ordinary 5 % CO₂ incubator is usable for the culture. The carbon dioxide concentration and culture temperature are not essential so far as they are within the ranges for the ordinary cell culture.

After the culture under the above-described conditions for about 1 week, colonies can be clearly recognized. At this time, the number of the cells constituting the colony is about 10 to about 30. The term "early stage" herein indicates such a stage. When the cells are cultured under the above-described conditions, the cell colony observed at this stage is mainly composed of the small hepatocytes and it is not yet completely surrounded by non-parenchymal cells.

ln the present invention, it is desirable to use small hepatocyte colonies free from cells other than the small hepatocytes, such as non-parenchymal cells including stellate cells and biliary epithelial cells, as far as possible. Specifically, it is desirable to use a small hepatocyte colony wherein at least about 70 % of the total number of cells constituting the colony are small hepatocytes. It is more desirable to use a colony wherein at least 80 %, in particular at least 85 %, of the total number of cells constituting the colony are small hepatocytes. The colony obtained by culturing the small hepatocytes isolated from the liver on the above-described medium under the above-described conditions, which is composed of about 10 to 30 cells, usually satisfies these conditions. Thus, the small hepatocyte colonies thus isolated at the early stage can be used for the method of preparing the liver tissue of the present invention.

The colonies thus formed are preferably detached from the culture container and transferred to a new medium by, for example, a non-enzymatic method. For example, it is preferred that the cells are non-enzymatically detached from the culture dish by using a metal chelating agent and various non-enzymatic dissociating agents. The metal chelating agents usable herein may be any of those having low cytotoxicity such as those generally used for the detachment of the adhesive cells, such as EDTA, EGTA and/or salts thereof, which may be used in an ordinary concentration for them respectively. Sodium salt of EDTA is particularly preferred and it is used in a concentration of preferably 0.01 to 0.05 % (w/v), more preferably 0.01 to 0.02 % (w/v). When sodium salt of EGTA is used, it is preferable to use it at about 0.5 mM. The treatment time may be as short as several seconds as in case of rinsing the cells, preferably about 30 seconds to about 10 minutes, more preferably about 1 minute to about 5 minutes. The treatment time is preferably as short as possible because the cells are injured by the long time treatment.

As the non-enzymatic cell dissociating agent, a solution obtained by adding EDTA, glycerol, sodium citrate, etc. to Hanks buffer solution free of Ca and Mg my be used. For example, a non-enzymatic cell dissociating agent named Cell dissociation solution by Sigma is commercially available. Specifically, for example, a phosphate buffer containing about 0.02 % of EDTA is poured on the cells. After leaving them to stand for several minutes, the EDTA solution is removed. A non-enzymatic cell detaching agent such as Cell dissociation solution is poured thereon and they are left to stand at, for example, 37°C for about 5 to 30 minutes, preferably about 10 to 15 minutes. For minimizing the damage of the cells, the time for treatment with the non-enzymatic dissociating agent is preferably as short as possible as in the treatment with the metal chelating agent. Then, the small hepatocyte-rich colonies are detached from the culture dish by the gentle pipetting. The small hepatocyte-rich colonies of the present invention can be extremely easily detached even without using any enzyme under those mild conditions. By isolating the colonies at the early stage by the above-described process, the contamination by other cells, particularly non-parenchymal cells, can be controlled at a lower level.

If necessary, the colonies are washed by repeatedly centrifuging and suspending, and they are finally suspended in a suitable medium or the like. Although the number of the cells and/or small hepatocytes per colony may be counted, if necessary, with a phase-contrast microscope, this step is usually unnecessary if the colonies are isolated at the early stage as described above. The number of the colonies can be determined with an image-analyzing program such as IPlab. The number of the cells constituting the colony can be determined with the phase-contrast microscope after immunostaining with a marker such as albumin or cytokeratin 8. It is also possible to determine the number of the cells over time by recording the image on a computer with a phase-contrast microscope and CCD camera.

A medium containing nicotinamide, vitamin C, growth factor, DMSO, etc. can be used for the successive subculture for keeping the growth of the cells as in the step of directly obtaining small hepatocytes from the liver. The concentration of each of nicotinamide, vitamin C, growth factor, DMSO, etc. is the same as that described above. Although the same medium is usable for the successive subculture for the formation of the liver tissue, the serum-free medium is preferred as described above for minimizing the contamination with protein unnecessary for liver tissue transplantation.

The small hepatocyte colony thus isolated in the early state and only containing non-parenchymal cells less than about 30 % can be cultured even in a medium substantially free of the growth factor because the dependency thereof on the growth factor is extremely lowered. Thus, it is one of the characteristic features of the small hepatocyte-rich colonies of the present invention that the growth factor is not indispensable for the growth. Another characteristic feature of the isolated small hepatocyte-rich colony of the present invention is that when the colony is successively subcultured in the above-described medium, the maturation scarcely occurs and the growth can be continued two-dimensionally because cells other than hepatocytes are small in number.

Thus, when the colony formed from the cells directly isolated from the liver, enriched with the small hepatocytes, is continued to be cultured, it can be grown without maturating. It is also possible to isolate a colony of the small hepatocytes grown by the above-described non-enzymatic method, to divide the colony into colonies each having 10 or less cells and continuing the successive subculture. The colonies thus subjected to the successive subculture can be cultured in the above-described medium until colonies each composed of about 10 to 30 cells are formed. The colonies each composed of about 10 to 30 cells thus obtained are also included in the small hepatocyte-rich colonies of the present invention. The colonies each composed of about 10 to 30 cells and obtained by repeating the successive subculture at least once are also included in the small hepatocyte-rich colonies of the present invention.

The maturation of the small hepatocyte-rich colonies which are mainly composed of the small hepatocytes, which are obtained by the culture as described above, can be induced to a stage extremely close to that of the hepatocytes *in vivo* by shortly culturing the colonies in the presence of extracellular matrix and then in a medium without extracellular matrix.

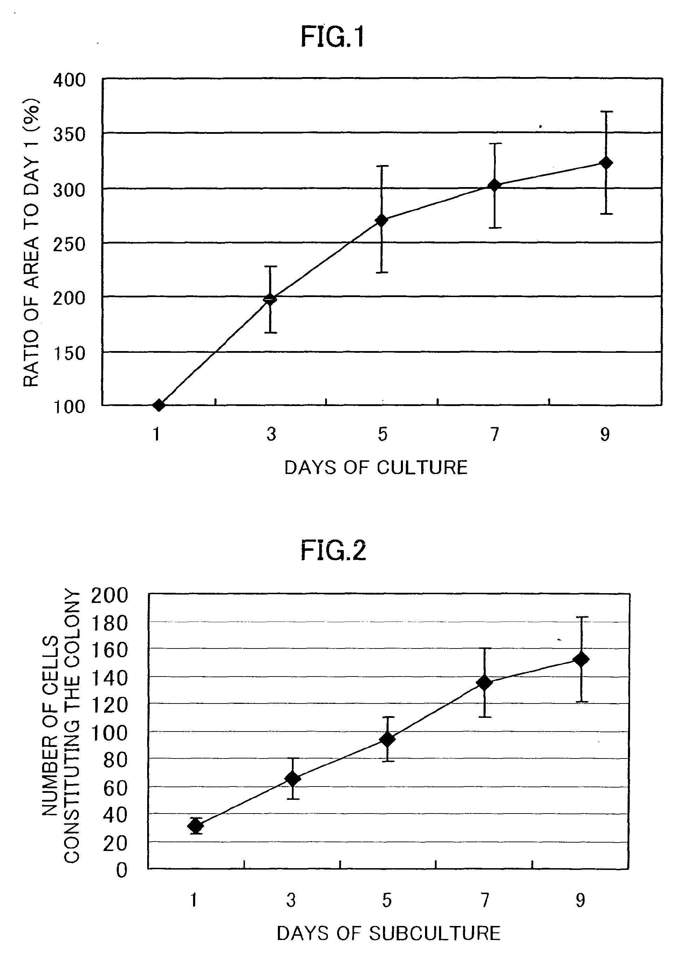

The above-described medium (serum-free medium) in the culture dish is seeded with the small hepatocyte-rich colonies in which the ratio of the small hepatocytes are at least about 70 % based on the total number of the cells, preferably the small hepatocyte-rich colonies composed of about 10 to 30 cells in which the ratio of the small hepatocytes are at least about 70 % based on the total number of the cells, at a density of 500 to 10,000 colonies/ml, preferably 1,000 to 4,000 colonies/ml, more preferably 1,500 to 3,000 colonies/ml so as to obtain 200 to 1,000 colonies/cm², preferably 250 to 500 colonies/cm². For inducing the maturation into the liver tissue, it is preferred that the density of the colonies in the culture dish is not too high. For example, after the culture for about 10 to 14 days, the small hepatocyte-rich colonies grow to an about 5 times as large area or to about 6 times as large number of cells (Figs. 1 and 2), and about 20 to 30 % of the bottom area of the culture dish is covered with the cells. As for the time of the addition of the extracellular matrix, the colonies at such a stage is particularly preferred. The induction of the maturation can be accelerated by adding 50 µg/ml to 1 mg/ml, preferably 100µg/ml to 1 mg/ml, of the extracellular substance containing laminin, collagen, various proteoglycans, entactin, fibronectin, etc. to the culture medium.

The maturation of the small hepatocyte colonies are remarkably developed by culturing the cells with the extracellular matrix of such a concentration for about 1 to 3 days, and then culturing them in the extracellular matrix-free medium. For example, from the next day when the cells are transferred into the extracellular matrix-free medium, the morphological change of the small hepatocytes is clearly observed. When the culture is continued in the extracellular matrix-free medium, the maturation of the small hepatocytes further remarkably proceeds, the colonies enlarge and mature to form the structure extremely close to that of the *in vivo* liver tissue. The extracellular matrix usable for the maturation of the small hepatocytes preferably contains laminin and type lV collagen, more preferably, it contains many of the basement membrane components (such as collagen, proteoglycan and laminine). For example, an extracellular matrix preparation (containing 60 % of laminine, 15 % of lV collagen, proteoglycan, entactin etc.) extracted from Engelbreth-Holm-Swarm sarcoma (EHS) or the extracellular matrix preparation commercially available under the trade name of Matrigel (Collaborative Biomedical Products) is particularly preferred.

The tissue thus formed *in vitro* extremely close to the *in vivo* liver tissue may be used not only for the various investigations of the liver function but also for the transplantation of the liver.

The liver tissue particularly suitable for the liver-tissue transplantation can be formed by placing the small hepatocyte-rich colonies of the present invention on a sheet made of a biocompatible material which is bioabsorbable or biodegradable. The term "bioabsorbable" herein indicates that the material is metabolized or incorporated into the cells mainly by the physiological action of the living body. The term "biodegradable" herein mainly refers to the property that the materials is decomposed by the physiological action of the living body. Both terms are herein not strictly distinguished from each other because they partially overlap. The term "biocompatible" herein indicates that the material is not significantly recognized as a foreign substance by living body.

The biocompatible and bioabsorbable sheet usable in the present invention preferably has a fiber structure. Namely, in the process of the present invention, the small hepatocyte-rich colonies of the present invention penetrate into the cavities in the fiber structure of the sheet and therefore, the concentration of the extracellular matrix secreted by themselves in the narrow cavities is increased to significantly stimulate the maturation. ln addition, because the contacting area between the cells is increased in the narrow space, the cubicity of the respective cells is increased and, as a result of those conditions, the liver tissue is formed. Thus, the sheet used in the process of the present invention preferably has a pleated structure.

The biocompatible and bioabsorbable sheets usable in the present invention include, for example, collagen sheets, collagen sponges and polyglycolic acid sheets. Commercially available materials usable for this purpose include, for example, absorbent collagen sheets sold under a trade name of Helistat by Integra Life Sciences Corporation and an absorbent polyglycolic acid felt sold under a trade name of Neoveil by Gunze Ltd.

When the small hepatocyte-rich colonies of the present invention are to be placed on the above-described sheet, a cell suspension adjusted to a relatively high concentration for minimizing the quantity thereof such as 500 to 10,000 colonies/ml, preferably 1,000 to 4,000 colonies/ml, more preferably 1,500 to 3,000 colonies/ml is added dropwise to the sheet in an amount of 200 to 1,000 colonies/cm², preferably 250 to 500 colonies/cm². Then they are left to stand until the colonies completely adhered to the sheet. The period must be enough for the colonies to adhere to the sheet, which is at least 20 minutes, preferably at least 30 minutes and particularly preferably at least 1 hour. Then a medium suitable for culturing the small hepatocytes is added thereto. This medium may be the medium used for isolating the small hepatocytes from the liver and it is preferably serum-free. Small hepatocyte culture medium II shown in Table 2 in Example is the medium suitable for culturing the small hepatocyte-rich colonies of the present invention on the sheet. After the culture carried out under such conditions, the small hepatocyte colonies on the sheet are remarkably matured and enlarged. Thus, about 10 to 15 days after, a structure very close to the *in vivo* liver tissue is formed on the sheet. The culture may be further continued to grow the liver tissue. However, because the bioabsorbable or biodegradable sheet is inclined to be dissolved in the culture mixture, it is preferred for the transplantation of the liver that the period of time of the culture is not longer than about 3 weeks.

The liver tissue particularly suitable for the transplantation can be formed also by placing the small hepatocyte-rich colonies on a sheet composed of a biocompatible, bioabsorbable or biodegradable material and then adding the extracellular matrix thereto. The conditions in this method such as the timing of the addition of the extracellular matrix, the concentration, the usable sheet and the number of the cells used for the maturation may be the same as those in the case of using the extracellular matrix and sheet separately. However, the concentration of the extracellular matrix may be suitably lowered. For example, the maturation can be accelerated by adding 100 to 1,000 mg/l of laminin, type lV collagen, Matrigel containing them or the like to the culture medium after the colonies have sufficiently adhered to the sheet or, in other words, within one hour after the colonies were placed on the sheet. It is enough that the extracellular matrix is added only once and then the hepatocytes may be cultured in the extracellular matrix-free culture medium.

The liver tissue thus formed on the sheet can be used as for the transplantation of the liver as a whole without separating the sheet.

The properties of the cells, as hepatocytes, in the colony matured by the addition of the extracellular matrix or by the culture on the sheet can be confirmed by analyzing the various markers. The markers usable are those which exhibit remarkable difference between the mature hepatocytes and the immature small hepatocytes such as those in the small hepatocyte-rich colonies immediately after the isolation. For example, various markers known as the hepatocyte-specific markers may be used.

The analysis can be conducted, for example, as follows: The cells are suspended in a suitable buffer such as Hepes buffer (10 mM Hepes, 0.25 M sucrose, 0.5 mM MgCl₂) and then they are mechanically homogenized with a micro syringe. The obtained cell lysate is divided into a cell membrane fraction, cytoplasm fraction and nucleus fraction by density-gradient centrifugation or the like, and proteins in each fraction can be analyzed by SDS-PAGE. Alternately, hepatocyte-specific markers such as albumin and fibrinogen secreted in the medium are analyzed by the same method as that described above.

Hepatocyte-specific markers which may be used as the index to the maturation of the small hepatocytes include, for example, HNF4, HNF6, C/EBPα. C/EBPβ and tryptophan dioxygenase (TO) or serine dehydrogenase (SDH) for the hormone induction expression, and transferrin, α₁-antitrypsin, fibrinogen and albumin.

Because the liver tissue formed on the sheet can be easily handled, this liver tissue is particularly suitable for the transplantation of the liver. In the transplantation, the culture medium-side of the sheet during the culture, namely, the side thereof on which the liver tissue is mainly formed, may be closely adhered to the organ and then fixed to the organ with a surgical stapler or the tissue and the organ are sutured. If necessary, an immunosuppressant such as FK 506 may be administered to the animals. Because the sheet is bioabsorbable, it is absorbed in the living body in about 1 to 2 weeks and the liver tissue on the sheet roots on the liver *in vivo.*

When the small hepatocyte-rich colonies induced to mature according to the process of the present invention are co-cultured with a chemical substance, the expression of a gene corresponding to the chemical substance, particularly, a drug metabolizing enzyme gene can be induced. For example, it can be supposed that a chemical substance which induces or represses a special drug metabolizing enzyme in the small hepatocyte-rich colonies, which has been induced to be matured, may also induce or represses the expression of the drug metabolizing enzyme gene in the liver *in vivo.* Further, it was found that in the *in vitro* mutagenesis tests of a chemical substance capable of attaining the mutation as a result of the metabolism with a specified drug metabolizing enzyme, the expression of the drug metabolizing enzyme is induced by a specified chemical substance in the small hepatocyte-rich colonies which have been induced to mature, and then a chemical substance to be tested can be added to the small hepatocyte-rich colonies.

The term "drug-metabolizing enzyme" generally indicates a group of enzymes which catalyze the reactions such as oxidation, reduction and addition reactions of various chemical substances in the liver and which are historically widely recognized by those skilled in the art as enzyme groups concerning so-called "detoxification reaction" which are also called "liver-drug metabolizing enzyme".

The liver tissue obtained from the small hepatocyte-rich colonies by inducing the maturation may be used for estimating effects of a chemical substance, particularly the effects thereof related to the liver function by co-culturing the liver tissue together with a given chemical substance and then examining the effect of the chemical substance on the specified gene expression in the small hepatocyte-rich colonies, in particular, their capacity of inducing or repressing the expression of the drug-metabolizing enzyme genes. The term "effect related to the liver function" involves the direct effects on the function of normal liver such as the induction or repression of the expression of the drug-metabolizing enzymes and also secondary *in vivo* effects due to the products generated as a result of the metabolism by the normal liver function such as the effects on the cells or tissues of organs other than the liver by the products. For example, the effects include the secondary where ethanol induces CYP2E1 in the liver and CYP2E1 thus produced forms a carcinogen in the body of a heavy smoker.

As described above, the drug-metabolizing enzymes act on exogenous and endogenous chemical substances such as carcinogens, food additives, insecticides, steroids, eicosanoids such as prostaglandins, lipids or lipophilic vitamins, in particular, substances having a relatively low polarity and substances generally called "lipophilic substances", and the enzymes are involved in the process where the substances are hydroxylate to obtain polarity, returned into the blood and excreted in the form of urine or they are excreted in the bile and thereby removed from the body. The drug-metabolizing enzymes include cytochrome P450 isozymes (such as CYP1A1, CYP2B1, CYP3A2, CYP2E1 and CYP4A1) concerning phase I and also those concerning phase 11 such as enzymes for adding glucuronic acid, sulfuric acid, acetates, glutathione and amino acids and methylation enzymes, e. g. glutathione S-transferase (GST), γ-glutamyl transferase (GGT), glucuronosyl transferase, acetyl transferase and ethyl transferase. Also in the present invention, the existence or non-existence of the induction or repression of these drug-metabolizing enzyme genes may be used for estimating the effect of a given drug. When one or more drugs are simultaneously administered, the simple effect or combined effects thereof on the living body can be estimated. For example, when a new drug is to be administered to a patient who has been treated with a specified drug, the propriety of the administration of the new drug and increase or decrease of the dose thereof can be determined *in vitro.* Further, the influences of administrating a particular drug on the habit of the patient can be estimated. Thus, the gene the expression of which is to be examined in the present invention is particularly preferably the gene encoding the drug-metabolizing enzyme (drug-metabolizing enzyme gene).

Because it is considered that about 50 % of chemical substances ingested by human beings are metabolized by cytochrome P450 enzyme family, various isozymes of cytochrome P450, such as CYP1A1, CYP2B1, CYP3A2, CYP2E1 and CYP4A1, are particularly suitable drug-metabolizing enzymes to be used for examining the induction or non-induction in the present invention. Such isozymes of cytochrome P450 are not so strongly expressed in ordinary tissues, and the isozymes corresponding to a particular chemical substance are remarkably induced when the chemical substance is administered. Those cytochrome P450 isozymes are thus suitable for the process of the present invention from the viewpoint of the facility of the tests.

Chemical substances the effect of which can be estimated by the process of the present invention are not particularly limited. However, they are preferably those considered to be easily incorporated into the cells by receptor-independent manners. For example, the chemical substances having a relatively low polarity are preferred, and lipophilic substances are more preferred. Further, in view of the convenience of the operation, substances which can be solubilized or easily dispersed in an aqueous medium are particularly preferred. Even when the chemical substance is a lipophilic substance, the substance is considered to have a relatively low concentration or to be solubilized or dispersed in an aqueous medium at the time when it reaches the liver *in vivo*. Therefore, for example, when a chemical substance is soluble in an aqueous medium to form a solution at a relatively low concentration, this substance is suitable for the process of the present invention even if it is a lipophilic substance. It is also possible that when the chemical substance is soluble in an organic solvent known to be not seriously harmful in the cell culture, such as ethanol, methanol or dimethyl sulfoxide in the art, the substance may be used by dissolved it in such organic solvent so that the final concentration of the organic solvent is in the range to be not seriously harmful to the cell culture as known in the art. Such chemical substances include alcohols such as ethanol, various aromatic hydrocarbon compounds, various hormones including steroids, carcinogenic substances such as methylcholanthrene, hypnotic drugs such as phenobarbital, antidepressant drugs, and antipyretic analgesics such as aminopyrine. These substances induce or repress the expression of the drug-metabolizing enzyme genes.

The term "repression of the expression" herein includes the case where the expression of a specified gene such as a drug-metabolizing enzyme gene, of which expression has been already induced, is decreased and also the case where the induction of the expression of a specified gene, for example a drug-metabolizing enzyme gene, is inhibited when a drug for inducing the expression of the gene is co-existent. The term "expression" of a gene includes both the expression at the transcriptional level and also at the translational level. Accordingly, in the present invention, when the gene product is increased due to the increase in the translational rate, it is considered that "the expression is induced" even if the amount of mRNA is not significantly changed.

In the process for estimating an effect of a chemical substances, the small hepatocyte-rich colonies, which have been matured according to the process of the present invention, are co-cultured with the chemical substance to be examined for their effect in various concentrations, and genes expressed in the cells in the matured small hepatocyte-rich colonies are then identified and/or the expression level thereof is determined to examine whether the expression of the particular genes, such as drug-metabolizing enzyme genes, are induced or not. Alternately, the small hepatocyte-rich colonies, which have been matured according to the process of the present invention, are co-cultured with a chemical substance, the effect of which is to be examined, also together with a specified chemical substance known to induce the expression of the genes such as those encoding the drug-metabolizing enzymes or, after inducing the expression of the above-described genes in the small hepatocyte-rich colonies matured according to the process of the present invention with such a chemical substance, the small hepatocyte-rich colonies in which the above-described expression of the drug-metabolizing enzyme has been induced are co-cultured with a chemical substance, the effect of which is to be examined, and the genes expressed in the cells in the colonies enriched in matured small hepatocytes are identified and/or the expression level thereof is determined to examine the presence or absence of the expression of the specified genes, such as drug-metabolizing enzyme genes, caused by the chemical substance.

The expression of the gene can be detected, identified and determined at a mRNA level or at a protein level. Accordingly, the identification of the gene expression induced or repressed in the above-described process and/or the determination of the gene expression level may be conducted by identifying and determining a specified protein such as the induced or repressed drug-metabolizing enzyme protein (examples of them are given above), or by identifying and determining drug-metabolizing enzyme mRNA. mRNAs and proteins can be identified and/or determined by an ordinary method known in the art. For example, mRNA can be identified and/or determined by Northern blot, and proteins can be identified and/or determined by SDS-polyacrylamide electrophoresis and Western blot. Antibodies specific to various drug-metabolizing enzymes are commercially available and, if necessary, they can be prepared by the ordinary methods known in the art.

### Examples

The present invention will be more easily understood from the following Examples and attached drawings. It is to be understood that those Examples and drawings are given for facilitating the understanding of the present invention, which should not be construed to limit the present invention.

### Example 1 Method of isolating small hepatocytes

### (1) lsolation of small hepatocytes from liver tissue

The liver of each mature rat (10 to 15 weeks old) was perfused through the portal vein according to Seglen's method using Hanks' solution free of Ca and Mg supplemented with 0.2 mM EGTA. After the perfusion with 40 ml/min of Hanks' solution for 4 minutes, the rat was perfused with 20 ml/min of Hanks' solution containing 0.02 % of collagenase (Yakult) for 10 minutes. The hepatocytes were collect into a beaker from the digested liver according to the conventional method. The cell suspension was filtered through a 70 µm mesh filter and then centrifuged at 50 x g for 1 minute. The supernatant was taken and centrifuged at 50 x g for 5 minutes. The precipitated cells were washed with a culture medium (Leibovitz L-15, 10 % fetal calf serum, 10⁻⁷ M dexamethasone, 0.5 µg/ml insulin and an antibiotic) and then centrifuged at 50 x g for 5 minutes. The similar process as above was repeated once, and the obtained mixture was further centrifuged at 150 x g for 5 minutes twice and then centrifuged at 50 x g for 5 minutes. The precipitated cells were suspended in a new culture medium to obtain the small hepatocytes. The number of the living cells was counted and then the cell density was controlled at 1.0 to 2.0 x 10⁵/ml.

### (2) Culture of small hepatocytes

About 6 x 10⁵ /culture dish of the cells produced in step (1) were plated in 60 mm culture dishes. After culturing them in an air incubator for 3 hours, the medium was exchanged. The culture medium used (small hepatocyte culture medium I) was based on Dulbecco modified Eagle's medium as shown in Table 1 given below. The cells were cultured in a 5 % carbon dioxide gas incubator at 37°C. The culture medium was exchanged about every other day. About one week after the start of the culture, colonies of the small hepatocytes could be clearly recognized.

**Table 1**

| Small hepatocyte culture medium I | |
|---|---|
| Dulbecco modified Eagle's medium | (GIBCO Laboratories) |
| + 20 mM HEPES | (Dojindo) |
| + 25 mM NaHCO₃ | (Katayama Chemical Co.) |
| + 30 mg/l L-proline | (Sigma Chemical Co.) |
| + 0.5 mg/l insulin | (Sigma Chemical Co.) |
| + 10⁻⁷ M Dexamethasone | (Sigma Chemical Co.) |
| + 10 %FBS | (Hyclone Laboratories, Inc.) |
| + 10 mM Nicotinamide | (Katayama Chemical Co.) |
| + 1 mM L-Ascorbic acid 2-phosphate | (Wako Pure Chemical Inc.) |
| + 10 µg/l EGF | |
| + Antibiotic | |
| (+ 1 % dimethyl sulfoxide (DMSO)* Aldrich) | |

| | |
|---|---|
| * The addition of DMSO was started at the time of the medium exchange on the 4^{th} day of the culture. | |

### Example 2 lsolation of small hepatocyte-rich colonies

When the small hepatocyte colonies had become to be clearly observed about 1 week after the culture of the small hepatocytes isolated as described in Example 1, the small hepatocyte colonies were prepared as described below.

The cells were washed twice with sterilized phosphate buffer. Then the cells were immersed in a phosphate buffer containing 0.02% EDTA. After leaving them to stand for several minutes, the phosphate buffer was aspirated. 1 ml of enzyme-free cell detaching solution (Cell dissociation solution, Sigma) was added into the culture dish and they were left to stand at 37°C for 15 minutes. Then the cells were gently pipetted so as not to damage the cells to carefully detach the cells from the culture dishes. The cells were collected in centrifugation tubes and then centrifuged at 50 x g for 5 minutes. The supernatant was aspirated, then washed with the culture medium containing 10 % of serum and then centrifuged again. The supernatant was aspirated and then a serum-free culture medium (small hepatocyte culture medium II) shown in Table 2 was added in various volumes to obtain cell suspensions. The concentration of the small hepatocyte colonies was determined. The concentration of the small hepatocyte colonies was 500 to 10,000/ml.

The total number of the cells constituting the small hepatocyte colony thus prepared was 18.5±9.4 as determined with a phase-contrast microscope. The occupation of the small hepatocytes in the small hepatocyte colony was about 70 to about 90 %.

**Table 2**

| Small hepatocyte culture medium 11 | |
|---|---|
| Dulbecco modified Eagle's medium | (GIBCO Laboratories) |
| + 20 mM HEPES | (Dojindo) |
| + 25 mM NaHCO₃ | (Katayama Chemical Co.) |
| + 30 mg/l L-proline | (Sigma Chemical Co.) |
| + 0.5 mg/l insulin | (Sigma Chemical Co.) |
| + 10⁻⁷ M Dexamethasone | (Sigma Chemical Co.) |
| + 10 mM Nicotinamide | (Katayama Chemical Co.) |
| + 1 mM L-Ascorbic acid 2-phosphate | (Wako Pure Chemical Inc.) |
| + 10 µg/l EGF | |
| + Antibiotic | |
| (+ 1 % dimethyl sulfoxide (DMSO)* (Aldrich) | |

| | |
|---|---|
| * The addition of DMSO was started on the first day of the culture. | |

### Example 3 Induction of small hepatocytes into liver tissue (1)

1.5 - 3.0 x 10³ colonies/dish of the small hepatocyte-containing colonies obtained in Example 2 were placed on a culture dish (diameter: 35 mm).

They were cultured in a 5 % carbon dioxide incubator at 37°C. About 1 week after the successive subculture, the colonies of the small hepatocytes produced colonies which have on average 3 times as large area as that on the first day and the number of the cells was increased to 5 times as large (Figs. 1 and 2).

When the area of the colonies at the bottom of the culture container had become about 20 % to about 30 % (about 20 % to about 30 % confluent) about 2 weeks after the successive subculture, 500 µg/ml of the extracellular matrix (product name: Matrigel) (composed of the basement membrane and containing 60 % of laminin, 15 % of type lV collagen, proteoglycan, entactin, etc.) extracted from Engelbreth-Holm-Swarm sarcoma (EHS sarcoma) was added to the culture medium.

After 2 days culture, the culture medium was exchanged with a Matrigel-free culture medium and then the culture was continued. The hepatocyte-specific markers were determined at intervals of 48 hours.

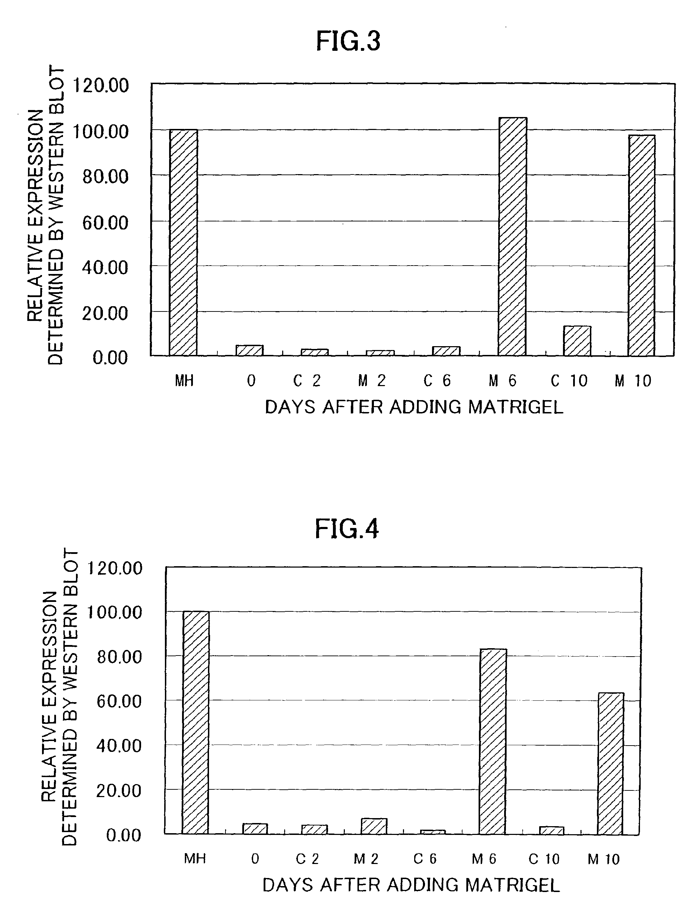

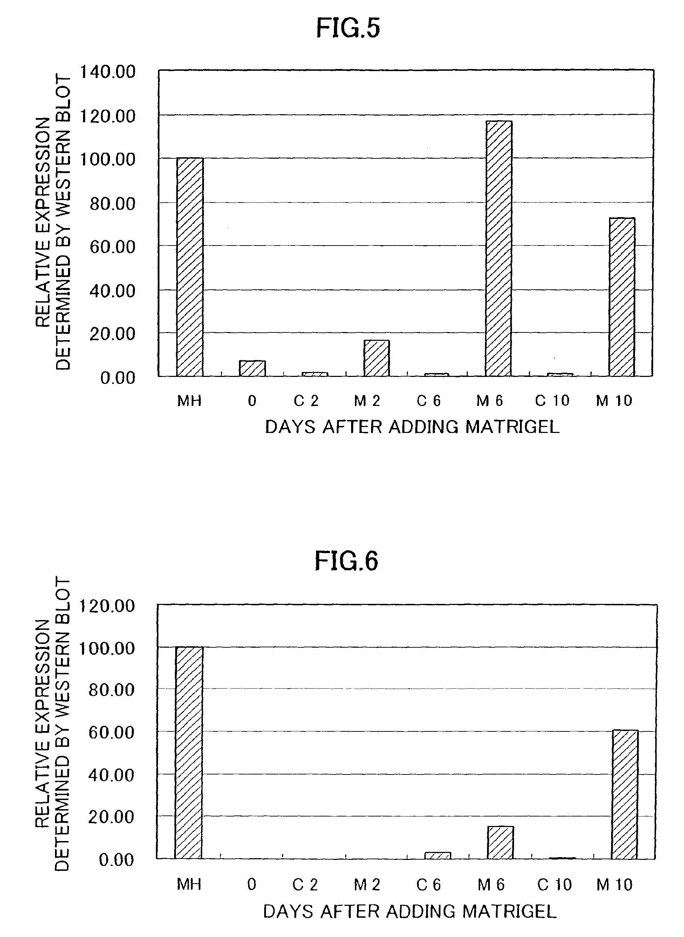

As a result, the production of C/EBPα and C/EBPβ, hepatocyte nucleus factor 4 (HNF4), hepatocyte nuclear factor 6 (HNF6), etc., which are the transcriptional factors considered to express only in the mature hepatocytes, was remarkably induced 2 to 10 days after the induction by Matrigel (Fig. 3 to 6). The induction of the production of those factors was confirmed by the electrophoresis of 20 µg of nuclear extraction protein, conducting Western blotting and determining the relative intensity of each band. Proteins from the mature hepatocytes (MH) were used as a positive control and proteins extracted from the Matrigel-free small hepatocyte-rich colonies were used as a negative control(C) (Figs. 3 to 6).

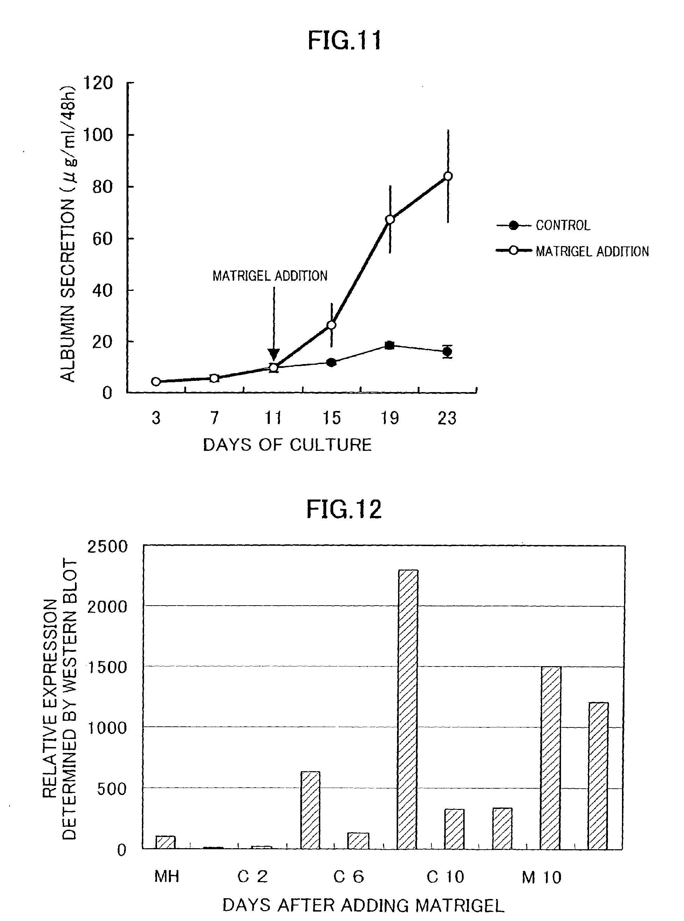

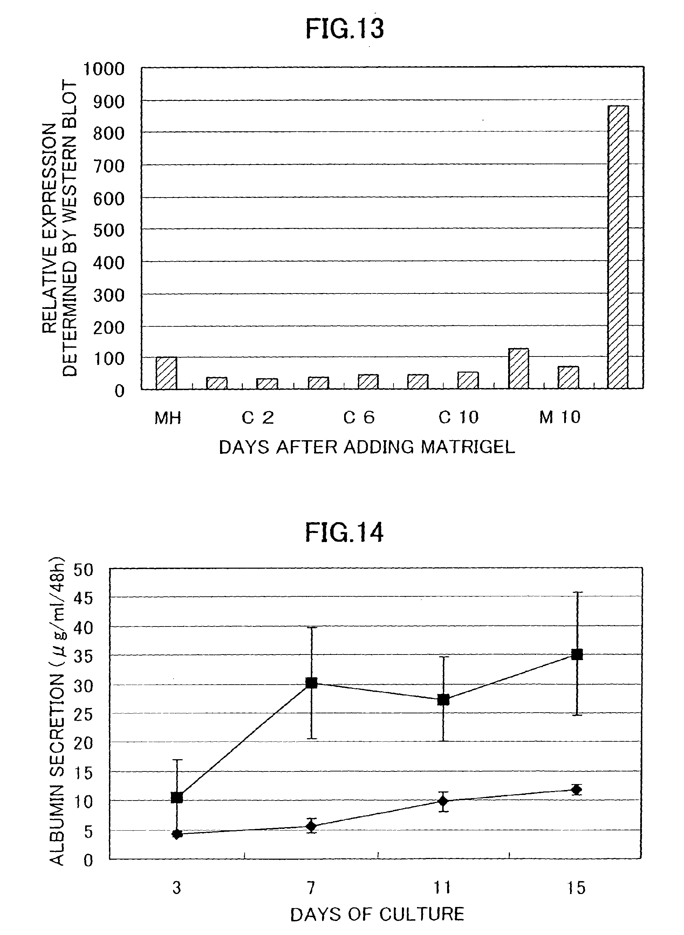

Further, the hormone-induced expressions of tryptophan 2-dioxygenase (TO) and serine dehydratase (SDH), of which expressions were considered to be controlled by those transcriptional factors, have become to be observed (Figs. 12 and 13). The induction was confirmed as follows: 500 µg/ml of Matrigel was added to the small hepatocyte-rich colonies and 10⁻⁵ M of dexamethasone and 10⁻⁷ M of glucagon were then added on the 9^{th} day after the start of the culture. After 24 hours the cells were collected and the proteins were extracted. 20 µg of the extracted proteins were analyzed by Western blotting to determine the intensity of each band for determining the relative intensity. Proteins from the mature hepatocytes (MH) were used as a positive control and proteins extracted from the Matrigel-free small hepatocyte-rich colonies were used as a negative control (C).

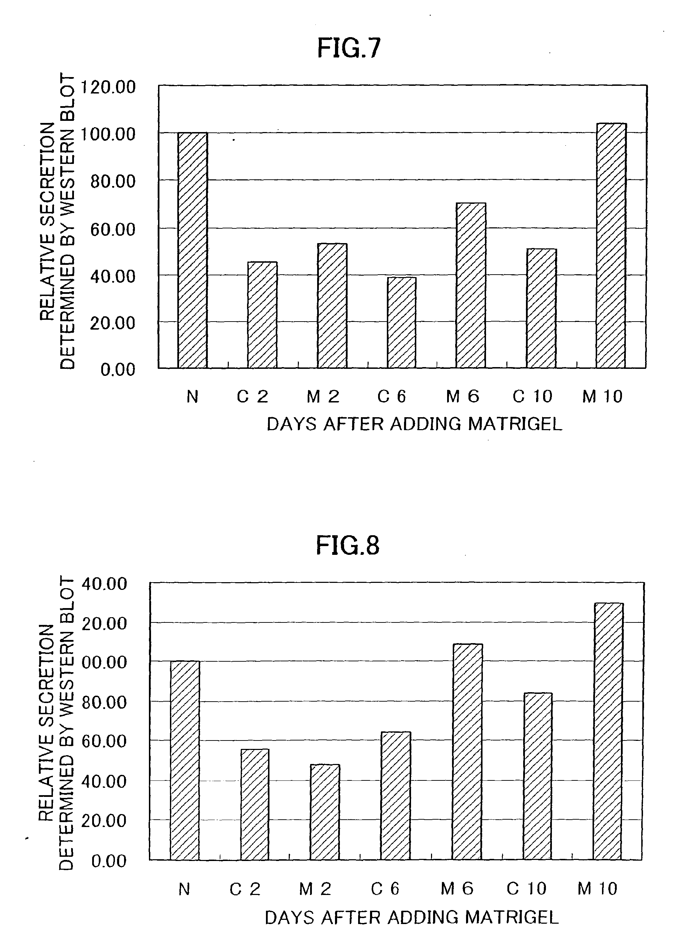

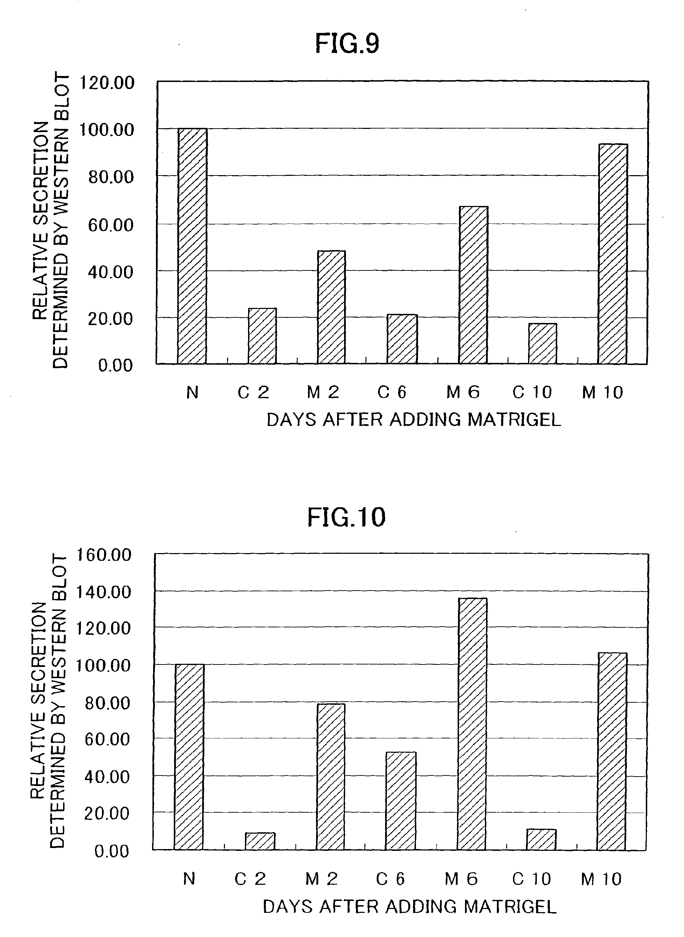

Further, the secretion of albumin, transferrin, α1-antitrypsin and fibrinogen was confirmed in the culture medium (Figs. 7 to 10). The secretion of these proteins was confirmed by the electrophoresis of 1 µl of the supernatant, Western blot and the determination of the relative intensity of each band. Normal rat serum or plasma was used as a positive control (N), and the supernatant obtained by culturing small hepatocyte colonies on a collagen-covered culture dish (C) was used as a negative control (Figs. 7 to 10).

The typical result, the detailed time course of the changes in the albumin secretion into the culture medium, is particularly shown in Fig. 11. These results indicate the maturation of the small hepatocytes or, in other words, the induction of the maturation of them into liver tissues is remarkably accelerated by the extracellular matrix.

When the small hepatocytes contacted the extracellular matrix, the volume of the cells rapidly increased and the cytoplasma thereof became larger. However, because the surface of the cells in contact with the culture dish is not significantly increased, the height of the cells was increased due to the increase in the volume and, as a result, the contact surface area between the adjacent cells to was increased. The cell junction is well developed between the cells, the cells bind by tight-junction and bile canaliculi were formed between the cells, and a bile-like secretion could be excreted in the bile canaliculi. In addition, the gap junction was well developed to improve the intercellular communication. In the cytoplasm, intracellular small organs such as mitochondria, Golgi apparatus and peroxisome developed well and glycogen granules became to be well observed. Those results indicate that the small hepatocytes can acquire the functions similar to those of the mature hepatocytes *in vivo.*

### Example 4 Induction of small hepatocytes into liver tissue (2)

The small hepatocyte colonies prepared in Example 2 were placed as a whole on the absorbable collagen sheet (trade name: Helistat) or on the absorbable polyglycolic acid felt sheet (trade name: Neoveil).

The small hepatocyte colony suspension prepared in Example 2 (1,500 to 3,000 colonies/ml) was slowly added dropwise on the above-described sheet having a size of 2.5 cm x 2.5 cm. A sufficient amount of the small hepatocyte colonies (total cell number: about 2,800 to 5,000) was placed on the sheet and they were then left to stand in 5 % CO₂ incubator at 37°C for about 1 hour. The culture medium shown in Table 2 was added to the sheet, and the culture was continued in 5 % CO₂ incubator at 37°C.

The small hepatocytes adhered to the sheet slowly grew, and a morphological transformation was observed on the sheet even on day 2 of the culture. Because the cells in the colonies in the gaps between the fibers of the sheet or those adhering on the fibers were brought into contact with the surrounding collagen fibers, the cells grew cubic and vigorously divided. The contact areas between the cells were increased because the number of the cells was increased in the narrow space. When the cell density had become proper, the cell division arrested and the cytoplasm became larger. Cell junctions were well developed between the cells and they were firmly attached by tight junction. Between the cells, bile canaliculi were formed and, in addition, a bile-like secretion could be excreted therein. Further, a gap junction was well developed and the intercellular communication was improved. The intracellular small organs such as mitochondria, Golgi apparatus and peroxisomes developed well and glycogen granules became to be well observed. Those results indicate that the small hepatocytes can acquire the functions similar to those of the mature hepatocytes *in vivo.*

A reason why the maturation can be induced even without covering the cells with an extracellular matrix is considered to be that the cells are settled by such physical factors.

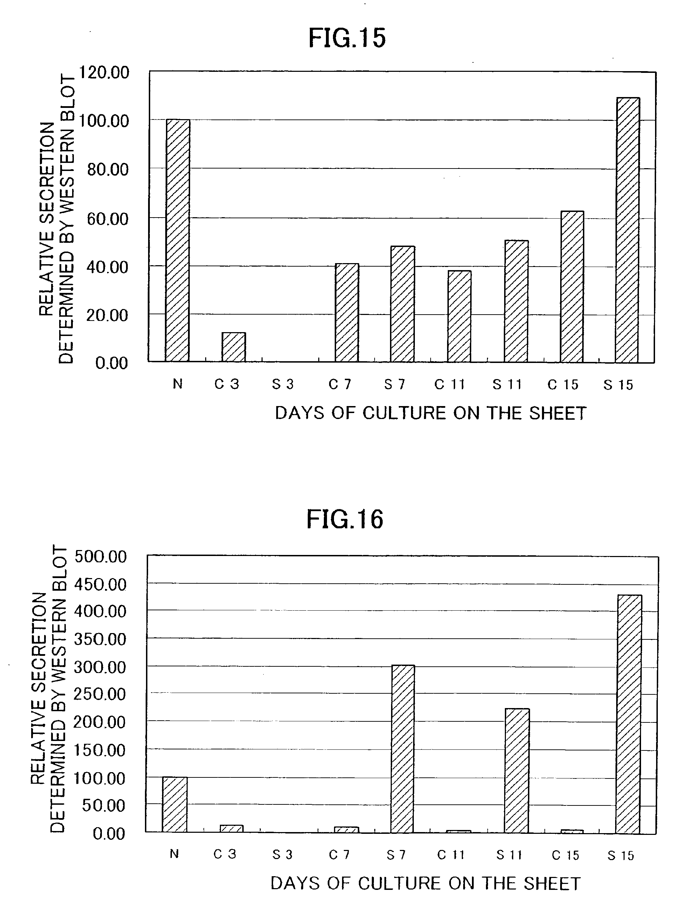

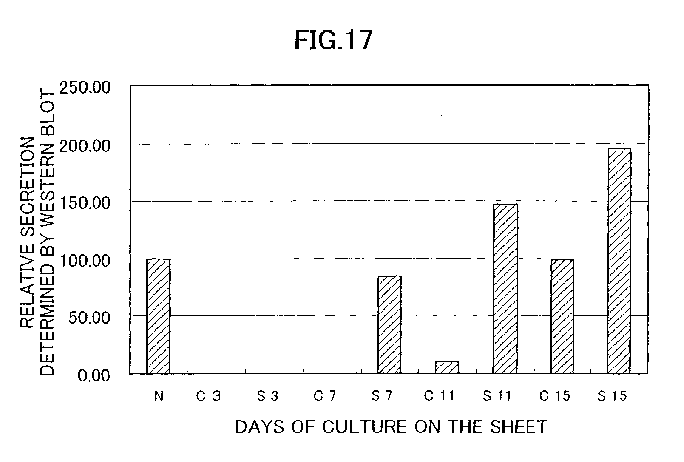

On day 7 to day 15 during the culture on the sheet, 1 µl of the culture mediumt was subjected to electrophoresis and then Western blot analysis and the relative intensity of each band was determined. As a result, it was found that the secretion of proteins characteristic to the matured hepatocytes such as albumin, transferrin, haptoglobulin and fibrinogen was remarkably increased (Figs. 14 to 17). In particular, the secretion of albumin was remarkable on day 2 of the culture and thereafter (Fig. 14).

In this experiment, it was again found that the maturation of the small hepatocyte colonies cultured on the collagen sheet was much more remarkable than that of the small hepatocyte colonies cultured on the collagen-coated culture dish.

### Example 5 Transplantation of liver tissue induced from small hepatocytes

The liver tissue derived from the small hepatocyte colonies formed by culturing the small hepatocytes on the collagen sheet for 14 days as described in Example 4 was transplanted together with the sheet to analbumin rats.

The abdomen of anesthetized rat was incised to expose the liver and about 2/3 of the liver was excised. The liver tissue formed on the sheet was placed, together with the sheet, on the remaining liver in such a manner that the culture medium side or, in other words the colony side, was placed on the organ. The sheet was anchored with a surgical stapler and the incised site was sutured. An immunosuppressant such as FK506 was given to the rat.

### Example 6 Determination of capacity of compounds in inducing hepatic drug-metabolizing enzymes using small hepatocytes

About 200 to 250 / cm² of the small hepatocyte colonies prepared in Example 2 were placed on a culture dish (diameter: 60 mm).

After the culture in a culture medium (1) (DMEM, 10 % FBS, 10 mM nicotinamide, 1 mM ascorbic acid 2-phosphate, 10 ng/ml EGF, 10⁻⁷ M dexamethasone, 0.5 µg/ml insulin, penicillin and streptomycin) for 12 to 24 hours, the culture medium was exchanged with a serum-free culture medium (2) (DMEM, 10 mM nicotinamide, 1 mM ascorbic acid 2-phosphate, 10 ng/ml EGF, 10⁻⁷ M dexamethasone, 0.5 µg/ml insulin, penicillin, streptomycin and 1 % DMSO). After the culture for additional 10 days while the culture medium was exchanged every other day, Matrigel diluted to 500 µg/ml with the culture medium was placed on the cells. 48 hours after the addition of Matrigel, the culture medium was exchanged, and the culture was continued by using DMSO-free culture medium (2) for 4 days. 6 days after the addition of Matrigel, drugs shown in the following Tables 3 to 6 were added in a concentration shown in the same Tables thereto, respectively. 24 hours after the addition of the drug, the cells were recovered.

Ethanol is a drug involved in the alcohol metabolism. Clofibrate is an antihyperlipidemic agent. Phenobarbital is a tranquilizing agent known to have a hepatic carcinogenic promoter. Pregnenolone is a precursor of progesterone which is a female luteal hormone, i.e. a kind of steroid hormones.

After the proteins were separated with 10 % SDS-PAGE, the intended protein was stained on the membrane by Western blot. The intensity of the band on the blot was digitized by a densitometer.

The primary antibodies used were goat anti-CYP2B1 antibody (diluted to 1/300), goat anti-CYP3A2 antibody (diluted to 1/300), goat anti- CYP2E1 antibody (diluted to 1/1000) and goat anti- CYP4A1 antibody (diluted to 1/1000) (all bought from Daiichi Kagaku Yakuhin). The secondary antibodies were HRP-conjugated anti-goat/sheep lg (diluted to 1/5000) bought from DAKO. The chromogenic substrate was Super Signal West Dura (chemiluminescence) purchased from Pierce.

Tables 3 to 6 show the relative activity of each enzyme as the induction capacity of each drug for the enzyme, wherein the activity of each enzyme in matured hepatocytes is defined as 100%. Pregnenolone was used in the form of a solution containing 0.5 % ethanol, and clofibrate was used in the form of a solution containing 0.5 % DMSO.

**Table 3**

| CYP2B1 induction capacity | | |
|---|---|---|
| Drug | Concentration (mM) | CYP2B1 induction capacity (%) |
| Phenobarbital | 0 | 30.8 |
| | 0.75 | 37.4 |
| | 1.5 | 47.9 |
| | 3.0 | 46.9 |
| | 4.5 | 55.0 |

**Table 4**

| CYP3A2 induction capacity | | |
|---|---|---|
| Drug | Concentration (mM) | CYP3A2 induction capacity (%) |
| Pregnenolone | Control (0.5 % ethanol) | 94.9 |
| | 0.5 x 10⁻³ | 115.5 |
| | 1.0 x 10⁻³ | 141.0 |
| | 2.0 x 10⁻³ | 148.1 |
| | 5.0 x 10⁻³ | 153.2 |

**Table 5**

| CYP2E1 induction capacity | | |
|---|---|---|
| Drug | Concentration (mM) | CYP2E1 induction capacity (%) |
| Ethanol | 0 | 74.1 |
| | 250 | 76.5 |
| | 500 | 82.4 |
| | 750 | 98.8 |
| | 1000 | 86.4 |

**Table 6**

| CYP4A1 induction capacity | | |
|---|---|---|
| Drug | Concentration (mM) | CYP4A1 induction capacity (%) |
| Clofibrate | Control (0.5 % DMSO) | 74.1 |
| | 0.1 | 76.5 |
| | 0.25 | 82.4 |
| | 0.5 | 98.8 |
| | 0.75 | 86.4 |

According to the present invention, small hepatocyte aggregates suitable for the preparation of the transplantable liver tissue can be easily prepared and, in addition, the maturation of the small hepatocyte aggregates into a liver tissue can be easily induced. ln particular, because the liver tissue formed on the bioabsorbable sheet from the small hepatocyte-rich colonies of the present invention can be transplanted to a vital liver, an injured liver can be easily repaired by the present invention. Further, the cell aggregates having a function very close to that of the matured liver tissue in the induction of a metabolic enzyme responding to a specified drug can be obtained. Therefore, according to the present invention, the drug having the specified function can be screened and also the effect of the drug can be estimated without conducting animal experiments.

### Alternative embodiments of the invention:

1. Small hepatocyte-rich colonies, in which at least 70 % of the total cells constituting each of the colonies are the small hepatocytes.
2. The small hepatocyte-rich colonies according to claim 1, wherein the total number of cells constituting each colony is 10 to 30.
3. A process for preparing small hepatocyte-rich colonies which comprises the steps of:
   (i) isolating hepatocytes from a liver,
   (ii) dividing the isolated hepatocytes into a heavy fraction enriched with parenchymal cells and a light fraction enriched with non-parenchymal cells but having a low parenchymal cell content, and recovering the light fraction,
   (iii) culturing the cells in the light fraction in a culture medium containing nicotinamide to form small hepatocyte colonies, and
   (iv) recovering the small hepatocyte colonies having a total number of cells of 10 to 30.
4. A process for preparing small hepatocyte-rich colonies, which comprises the steps of:
   (i) dissociating colonies from a culture dish by reacting an enzyme or without reacting an enzyme on small hepatocytes forming the colonies to recover the small hepatocytes,
   (ii) successively subculturing the recovered small hepatocytes by using a culture medium containing nicotinamide to form colonies each composed of 10 to 30 cells in total, and
   (iii) recovering the colonies, each composed of 10 to 30 cells in total.
5. A process for maturating small hepatocyte-rich colonies into a liver tissue, which comprises the steps of:
   (i) culturing the small hepatocyte-rich colonies according to claim 1 or 2,
   (ii) adding an extracellular matrix to the medium containing the cultured small hepatocyte-rich colonies and
   (iii) further culturing the small hepatocyte-rich colonies, which have been cultured in the extracellular matrix-containing medium, in an extracellular matrix-free medium.
6. The process for maturating small hepatocyte-rich colonies into a liver tissue according to claim 5, wherein the density of the small hepatocyte-rich colonies is 200 to 1,000 colonies/cm² at the initial stage of the culture.
7. A process for maturating small hepatocyte-rich colonies into a liver tissue, which comprises the steps of:
   (i) placing the small hepatocyte-rich colonies according to claim 1 or 2 on a bioabsorbable sheet, and
   (ii) placing the sheet in a culture medium to culture the small hepatocyte-rich colonies on the sheet.
8. The maturation process according to claim 7, wherein the density of the colonies on the sheet is 200 to 1,000 colonies/cm² at the initial stage of the culture.
9. A process for preparing a liver tissue for transplantation, which comprises the steps of:
   (i) placing the small hepatocyte-rich colonies according to claim 1 or 2 on a bioabsorbable sheet, and
   (ii) placing the sheet in a serum-free culture medium to culture the small hepatocyte-rich colonies on the sheet.
10. The process for preparing the liver tissue for transplantation according to claim 9, wherein the density of the colonies on the sheet is 200 to 1,000 colonies/cm² at the initial stage of the culture.
11. A method of estimating an effect of a chemical substance on a liver function *in vitro,* which comprises the steps of contacting the small hepatocyte-rich colonies matured according to the process of any one of claims 5 to 8 with the chemical substance, identifying a gene the expression of which is induced or repressed in the cells in the small hepatocyte-rich colonies, and/or determining the expression level thereof.
12. A method of estimating an effect of a chemical substance on a liver function *in vitro,* which comprises the steps of contacting the small hepatocyte-rich colonies matured according to the process of any one of claims 5 to 8 with a chemical substance, identifying a gene the expression of which is induced or repressed in the cells in the small hepatocyte-rich colonies, and/or determining the expression level thereof, comparing the induction or repression pattern of the gene the expression of which is induced or repressed with an induction or repression pattern of the gene associated with a chemical substance having a known effect, and estimating the effect of the chemical substance on the liver function *in vitro* which exhibits the induction or repression pattern similar to the pattern of the chemical substance having a known effect as the effect of the chemical substance placed in contact with the small hepatocyte-rich colonies.
13. The method according to claim 11 or 12, wherein the gene the expression of which is induced or repressed is a drug-metabolizing enzyme gene.
14. A method for determining whether a chemical substance induces or represses the expression of a drug-metabolizing enzyme in the liver, which comprises contacting the small hepatocyte-rich colonies matured by the process according to any one of claims 5 to 8 with a chemical substance and determining a capacity of the chemical substance to induce or repress the expression of the drug-metabolizing enzyme gene in the cells in the small hepatocyte-rich colonies.
15. The method according to claim 13 or 14, wherein the drug-metabolizing enzyme gene is the gene encoding cytochrome P450 enzyme.
16. The method according to claim 15, wherein the drug-metabolizing enzyme gene is selected from the group consisting of the enzymes encoding CYP2B1, CYP3A2, CYP2E1 and CYP4A1.
17. A method of estimating the effect of a chemical substance connected with the liver function *in vitro*, which comprises the steps of placing the small hepatocyte-rich colonies matured according to the process of any one of claims 5 to 8 together with a chemical substance, identifying a gene the expression of which is induced or repressed in the cells in the above-described small hepatocyte-rich colonies, and/or determining the expression level thereof.

## Claims

1. A process for maturing small hepatocyte-rich colonies into a liver tissue, which comprises the steps of:
(i) culturing the small hepatocyte-rich colonies, wherein at least 70% of the total cells constituting each of the colonies are the small hepatocytes;
(ii) adding an extracellular matrix to the medium containing the cultured small hepatocyte-rich colonies;
(iii) culturing the small hepatocyte-rich colonies in an extracellular matrix containing medium; and
(iv) further culturing the small hepatocyte-rich colonies, which have been cultured in the extracellular matrix-containing medium, in an extracellular matrix-free medium.

2. The process according to claim 1, wherein the total number of cells constituting each small hepatocyte-rich colony is from 10 to 30.

3. The process according to claim 1 or 2, wherein the density of the small hepatocyte-rich colonies is from 200 to 1000 colonies/cm² at the initial stage of the culture.

4. A process for maturating small hepatocyte-rich colonies into a liver tissue, which comprises the steps of:
(i) placing the small hepatocyte-rich colonies on a bioabsorbable sheet, wherein at least 70% of the total cells constituting each of the colonies are the small hepatocytes; and
(ii) placing the sheet in a culture medium to culture the small hepatocyte-rich colonies on the sheet.

5. The process according to claim 4, wherein the total number of cells constituting each small hepatocyte-rich colony is from 10 to 30.

6. The process according to claim 4 or 5, wherein the density of the small hepatocyte-rich colonies is from 200 to 1000 colonies/cm² at the initial stage of the culture.
